# EUROPEAN PATENT APPLICATION

(11) **EP 3 831 895 A1**
(43) Date of publication of application: **09.06.2021**
(21) Application number: 19460063.1
(22) Date of filing: 02.12.2019
(51) Int. Cl.: C09D 5/03, A23G 3/34, A23P 20/00, A61K 9/28, C09D 7/61, C09D 7/40

(54) **THE USE OF MAGNESIUM PHOSPHATE AND A COLORING COMPOSITION**

(71) Applicant: Kiska Tomasz, 05-270 Marki (PL); Chudy, Zbigniew, 05-120 Legionowo (PL)
(72) Inventor: Kiska Tomasz, 05-270 Marki (PL); Chudy, Zbigniew, 05-120 Legionowo (PL)
(74) Representative: Padée, Grazyna

(57) **Abstract**

The invention relates to the use of magnesium phosphate as a colouring and opacifying agent in coating compositions for pharmaceuticals, dietary supplements and food products. The invention also relates to a coating composition comprising a film-forming substance at an amount from 20 wt % to 99.5% wt % and magnesium phosphate as a white dye at an amount from 0.5 wt % to 80 wt %. The composition may also contain additional excipients, such as plasticisers, fillers, anti-adhesive substances, anti-caking agents, additional opacifiers, dyes other than white.

## Description

The invention relates to a colouring composition in its dry form and its use in lacquers intended for coating various pharmaceutical forms and foodstuffs.

The application of a colour to the surface of food, pharmaceutical products or dietary supplements usually takes place in the process of covering the surface of tablets, capsules, pellets and other products with an appropriate coating layer, so-called varnish. Lacquers usually contain film-forming polymers, fillers, plasticisers, opacifiers, anti-caking agents, dyes and other known excipients. The varnish for coating is obtained by making an aqueous suspension of a previously prepared mixture of dry raw materials. Titanium dioxide (titanium white) is most often used as the dye that gives the coating a white colour. It is an additive widely used in dietary supplements, medicinal products, sweets all over the world.

Because of literature reports on the toxicity of titanium dioxide, some countries have recommended the withdrawal of this product from the ingredients of food and pharmaceutical products. The regulation on the ban was issued on April 17, 2019 in France, based on the qualifications of the International Agency for Research on Cancer (IARC), which classifies titanium dioxide as a carcinogen from group 2B, which means that it is "possibly carcinogenic to humans". The French Agency for Health and Safety ANSES has stated that the data confirming the safety of this additive is insufficient. Based on animal studies it was found that titanium dioxide nanoparticles can increase colitis. Therefore, the possibility and reasonableness of the future use of titanium dioxide in the pharmaceutical and food industries is uncertain.

Restrictions on use of titanium dioxide are prompting the search for new additives that could replace titanium dioxide as a white dye, which can also act as an opacifier in order to obtain a colour other than white. Such a product would have to be safe and approved for use in the coating process of dietary supplements, medicinal products, confectionery products and other products intended to be consumed.

Non-toxic white substances approved for use in the pharmaceutical and food industry, such as calcium carbonate, magnesium carbonates and magnesium oxide are known. However, these dyes do not provide the desired white colour when applied to tablets, pellets or foodstuff. Attempts to apply the above-mentioned substances as dyes for coating pharmaceutical and food products have shown a number of problems. First of all, these substances are unstable, turn yellow when exposed to light and during a longer storage. Secondly, they do not provide satisfactory parameters in terms of covering power after the varnish has dried on the surface. Thirdly, the listed substances do not have the right shade of white, and used as opacifiers for other dyes, they do not guarantee adequate colon saturation.

It should also be considered that in case of colouring compositions, the mechanical strength of coatings is very important. Products, such as tablets covered with a coating, are in fact exposed to abrasive forces during the subsequent production process. Colour durability is also very important because of the long shelf life of coated products, such as dietary supplements and pharmaceutical products. An important aspect when coating pharmaceutical and food products is the use of a dye that would not show properties of migration to the product core, e.g. to the tablet core.

One of the substances approved for use in the pharmaceutical industry is magnesium phosphate. It is a white and non-toxic substance. Magnesium phosphate is indicated for use in pharmaceutical products as an anti-caking and thickening agent. However, it has not been used to provide white colour to the coating of pharmaceuticals and foodstuffs.

The specification of U.S. Patent 3,960,611 discloses the use of magnesium phosphate in an active pigment composition, however the composition was intended for use in anti-corrosive paints.

A coating composition containing tribasic calcium phosphate and other known substances is known from the specification of WO2009024992 patent application. Calcium phosphate is added to the composition to reduce the tackiness of polyvinyl alcohol used as a film-forming polymer. The compositions according to this application contain dyes.

The invention was aimed to propose a new, safe white dye and to develop a new, safe colouring composition intended to provide white colour to pharmaceutical agents and foodstuffs.

Therefore, the subject of the invention is the use of magnesium phosphate as a colouring and opacifying agent in coating compositions for pharmaceuticals, dietary supplements and food products.

Preferably, magnesium phosphate with a fineness from 2 to 100 µm is used, and the magnesium phosphate composition is used to achieve a weight gain of the coated product from 3 wt % to 7 wt %.

Preferably, magnesium phosphate of formula Mg₃(PO₄)_{2·}xH₂O (tribasic magnesium phosphate) is used. Magnesium phosphate with various proportions of water molecules can be used.

Preferably, magnesium phosphate is used in a composition comprising this magnesium phosphate in an amount from 0.5 wt % to 60 wt %, and a film-forming substance selected from: polyvinyl alcohol (PVA), hydroxypropyl methylcellulose (HPMC), hydroxypropyl cellulose (HPC), agar, xanthan gum, acacia gum, shellac, gelatin, polyvinylpyrrolidone (PVP), sodium carboxymethyl cellulose, ethyl cellulose, sodium starch gluconate, other starch and cellulose derivatives, pectins or their derivatives, polymers of methacrylic acid in an amount from 40 wt % to 95.5 wt %, wherein a proportion of composition components refers to the dry weight of the composition.

Preferably, magnesium phosphate is used in a composition comprising, in addition to magnesium phosphate and a film-forming substance, also at least one known excipient selected from: plasticisers, fillers, anti-adhesive substances, anti-caking agents, additional opacifiers, dyes other than white.

Preferably, magnesium phosphate is used in the composition additionally containing a plasticizer selected from the group comprising: MTCs, vegetable oils, polysorbates (tweens), fatty acids, polyethylene glycols, sorbitol, glycerin, propylene glycol, trimethylaminoethanolamine, triacetin, triethyl citrate, lecithin, or combinations of these substances, in an amount from 0.5 wt % to 20 wt %.

Preferably, magnesium phosphate is used in the form of a suspension in water of the above-mentioned magnesium phosphate containing composition. Preferably the aqueous suspension is obtained by adding from 10 to 25 wt % of the composition to water.

Magnesium phosphate is used in compositions for coating medicinal products, foodstuffs and dietary supplements in the form of tablets, pellets, dragees and similar forms of pharmaceuticals, foodstuffs and dietary supplements requiring coating.

The invention also relates to a colouring composition for use in the coating process of pharmaceuticals, dietary supplements and food products. According to the invention, the colouring composition in its dry form contains, as the white dye, magnesium phosphate with a fineness from 2 to 100 µm in an amount of 0.5 wt % to 80 wt %, and further contains a film-forming substance selected from: polyvinyl alcohol (PVA), hydroxypropyl methylcellulose (HPMC), hydroxypropyl cellulose (HPC), agar, xanthan gum, acacia gum, shellac, gelatin, polyvinylpyrrolidone (PVP), sodium carboxymethyl cellulose, ethyl cellulose, sodium starch gluconate, other starch and cellulose derivatives, pectins or their derivatives, polymers of methacrylic acid in an amount from 40 wt % to 95.5 wt %.

The composition according to the invention may additionally contain known excipients selected from: plasticisers, fillers, anti-adhesive substances, anti-caking agents, additional opacifiers, dyes other than white.

Preferably, the plasticiser is selected from the group consisting of: MTCs, vegetable oils, polysorbates (tweens), fatty acids, polyethylene glycols, sorbitol, glycerin, propylene glycol, trimethylaminoethanolamine, triacetin, triethyl citrate, lecithin, or combinations of these substances. Preferably, the proportion of a plasticiser in the composition is from 0.5 wt % to 20 wt %.

The selection of a suitable film-forming substance and possibly other optional excipients will be made by a person skilled in the art based on their knowledge. For example, a person skilled in the art will use polymers of methacrylic acid or phthalic derivatives of cellulose to form a coating of pH-dependent products.

Magnesium phosphate in the composition according to the invention also acts as an opacifier in case of the addition of a dye other than white, but it is also possible to use an additional opacifier such as calcium carbonate, microcrystalline cellulose, starch, mica and other dispersing substances.

As a filler, the composition may contain dextrins, sugars, polyhydric alcohols in solid form, or combinations of these substances.

Dyes other than white can be synthetic dyes or their lakes, natural dyes or their lakes, vitamins.

As anti-caking agents, the composition may preferably contain talc, mica, starch or combinations of these substances.

The above excipients have been indicated only as examples as being preferred. However, this does not mean that the composition cannot contain other excipients known to those skilled in the art. The proportion of excipients in the composition is known to those skilled in the art. A person skilled in the art will choose the ingredients of the composition according to the needs.

The composition according to the invention with magnesium phosphate, and especially magnesium triphosphate, does not stratify, and after drying, it forms a continuous coating of white colour on the product surface. The composition has a good covering power, and in case when the coating has a colour other than white as a result of the addition of a second dye, the proportion of this second dye can be reduced up to five times without any degradation in colour quality. Thanks to this, the negative effect of dyes on the body is reduced.

The coatings obtained from the compositions of the invention are stable in accelerated ageing tests. Studies have confirmed the resistance of the obtained colour to light, and this resistance is maintained over a four-year storage period.

The coatings obtained using the compositions according to the invention are characterised by a friability below 0.01%, and in addition, they do not react with substances contained in the tablet core, e.g. acids or other pH-dependent raw materials.

The compositions of the invention are prepared by mixing the ingredients and then homogenizing them in a calibrator sized 40 mesh or in an impact mill. The homogeneous mixture can also be dissolved and then spray dried.

The composition according to the invention is applied to pharmaceutical products, dietary supplements or food products by a method known to those skilled in the art and selected according to the product. For example, tablets can be coated in a dragee drum, in a perforated coating drum, in a fluid-phase coating apparatus.

The invention is further illustrated in the examples.

### Example 1.

Coating compositions of the following composition were prepared. Tribasic magnesium phosphate Mg₃(PO₄)₂·xH₂O, with a particle size of 44 µm, was used in all compositions. To prepare the composition, the ingredients were mixed and then homogenised in a calibrator sized 40 mesh, and then ground in an impact mill.
Composition 1:
   HPMC - 45%
   magnesium phosphate - 32%
   polyethylene glycol - 13%
   microcrystalline cellulose - 10%
Composition 2:
   HPMC - 49%
   titanium dioxide - 25%
   polyethylene glycol -16%
   microcrystalline cellulose - 10%
Composition 3:
   hydrolysed PVA - 40%
   magnesium phosphate - 30%
   polyethylene glycol - 15.2%
   talc - 14.8%
Composition 4:
   hydrolysed PVA - 40%
   titanium dioxide - 25%
   polyethylene glycol - 18.2%
   talc - 16.8%
Composition 5:
   HPMC - 50%
   magnesium phosphate - 32%
   stearic acid - 7%
   talc - 7.9%
   triethyl citrate - 3%
   Tween 80 - 0.1%
Composition 6:
   HPMC - 50%
   titanium dioxide - 25%
   stearic acid 7%
   talk - 14.9%
   triethyl citrate - 3%
   Tween 80 - 0.1%
Composition 7:
   HPMC - 70%
   magnesium triphosphate - 30%
Composition 8:
   HPMC - 70%
   titanium dioxide - 30%

Each of the above-mentioned compositions was added to water in an amount of 17 wt %, followed by stirring at 25°C for about 1 hour to obtain a suspension.

Each of the compositions was applied on dark core tablets using the following process: The tablet bed was heated to 38-45°C. Air pressure in coating guns from 2.0 to 3.2 bar, nozzles with a diameter of 1.2 mm were used. Coating was conducted with a flow of drying air at a temperature from 50 to 65°C. The spraying rate was maintained so that the tablet bed was maintained at a temperature from 40 to 47°C. A process was carried out to achieve from 1 to 7 wt % weight gain of coated tablets.

After achieving adequate weight gain of the coated tablets, the tablets were cooled and discharged from the drum. The coated tablets were left for 2 hours in an open container to achieve equilibrium moisture content with the surroundings.

### Example 2.

Covering power, coating shades and mechanical strength of the obtained coatings were tested. Covering power and shade were determined visually by comparing to a standard. The intensity was classified on a five-point scale from 0 to +5. The value of zero means the uncoated, dark tablet core. The value of +5 refers to the white Pantone 000C standard.

The method determining the quality of the coating consisted of the mechanical abrasion of coated tablets. Friability was determined by measuring the weight loss of the coated tablets after intensive abrasion, according to the European Pharmacopoeia 2.9.7 "Friability".

The test results are shown in Table 1.

**Table 1.**

| Weight gain of the tablets: | Composition No.: | Covering power: | Friability: |
|---|---|---|---|
| 1% | 1 | 0 | 0.10% |
| | 2 | 0 | 0.08% |
| | 3 | 0 | 0.09% |
| | 4 | 0 | 0.09% |
| | 5 | 0 | 0.07% |
| | 6 | 0 | 0.09% |
| | 7 | 0 | 0.09% |
| | 8 | 0 | 0.09% |
| 2% | 1 | 0 | 0.05% |
| | 2 | 1 | 0.06% |
| | 3 | 1 | 0.05% |
| | 4 | 3 | 0.04% |
| | 5 | 0 | 0.03% |
| | 6 | 2 | 0.06% |
| | 7 | 0 | 0.05% |
| | 8 | 1 | 0.05% |
| 3% | 1 | 3 | 0.01% |
| | 2 | 5 | 0.01% |
| | 3 | 2 | 0.01% |
| | 4 | 5 | 0.01% |
| | 5 | 3 | 0.01% |
| | 6 | 5 | 0.01% |
| | 7 | 3 | 0.01% |
| | 8 | 5 | 0.01% |
| 4% | 1 | 4 | 0.01% |
| | 2 | 5 | 0.00% |
| | 3 | 5 | 0.00% |
| | 4 | 5 | 0.00% |
| | 5 | 3 | 0.01% |
| | 6 | 5 | 0.00% |
| | 7 | 3 | 0.01% |
| | 8 | 5 | 0.01% |
| 5% | 1 | 4 | 0.00% |
| | 2 | 5 | 0.00% |
| | 3 | 5 | 0.00% |
| | 4 | 5 | 0.00% |
| | 5 | 4 | 0.00% |
| | 6 | 5 | 0.00% |
| | 7 | 5 | 0.01% |
| | 8 | 5 | 0.01% |
| 6% | 1 | 5 | 0.00% |
| | 2 | 5 | 0.00% |
| | 3 | 5 | 0.00% |
| | 4 | 5 | 0.00% |
| | 5 | 4 | 0.00% |
| | 6 | 5 | 0.00% |
| | 7 | 5 | 0.00% |
| | 8 | 5 | 0.01% |
| 7% | 1 | 5 | 0.00% |
| | 2 | 5 | 0.00% |
| | 3 | 5 | 0.00% |
| | 4 | 5 | 0.00% |
| | 5 | 5 | 0.00% |
| | 6 | 5 | 0.00% |
| | 7 | 5 | 0.00% |
| | 8 | 5 | 0.01% |

As it has been shown, white colour with adequate covering power can be obtained by using magnesium phosphate instead of titanium dioxide, applying a larger weight gain of tablets, from 3 to 7 wt %. The value of the weight gain depends on the assumed colour intensity and brightness of the tablet core.

The obtained tablets were packed in PVC/A1 blisters and they were tested for stability in the following conditions:

| Test type | Storage conditions | Test duration |
|---|---|---|
| Long-term | 25°C±2°C, 60%±5% RH | 3 years |
| Intermediate | 30°C±2°C, 65%±5% RH | 18 months |
| Accelerated | 40°C±2°C, 75%±5% RH | 12 months |

All samples proved stable.

### Example 3.

Coating compositions having the following composition, in which dyes providing a colour other than white to the tablets were used, were prepared using the method of Example 1. For each dye, a composition containing magnesium phosphate as an opacifier and a comparative composition containing titanium dioxide with the same dye were prepared. Each of the compositions was coated on dark core tablets using the method described in Example 1.

The composition of the compositions, which allowed for the colour of the same intensity was compared below.

### Colour Pantone 549

*Composition 1a*
   HPMC - 45%
   magnesium phosphate - 32%
   triethyl citrate - 2.5%
   microcrystalline cellulose - 9.84%
   Tween 80 - 0.1%
   talc - 10%
   Brilliant Blue lake - 0.5%
   black iron oxide - 0.06%
*Composition 1b*
   HPMC - 47%
   titanium dioxide- 12%
   triethyl citrate - 4%
   microcrystalline cellulose - 14.4%
   Tween 80 - 0.1%
   talc - 20%
   Brilliant Blue lake - 1.2%
   black iron oxide - 0.3%

### Colour Pantone 300

*Composition 2a*
   HPMC - 47%
   magnesium phosphate- 25%
   PEG 6000 - 7.0%
   microcrystalline cellulose - 8.3%
   Tween 80 - 0.2%
   talc - 10%
   Brilliant Blue lake - 0.5%
*Composition 2b*
   HPMC - 40%
   titanium dioxide - 7%
   PEG 6000 - 15%
   microcrystalline cellulose - 10%
   poly dextrose - 13%
   talc - 10%
   Brilliant Blue lake - 5%

### Colour Pantone 107

*Composition 3a*
   HPMC - 47%
   magnesium phosphate - 35%
   triethyl citrate - 3%
   microcrystalline cellulose - 4.1%
   talc - 10%
   riboflavin - 0.9%
*Composition 3b*
   HPMC - 47%
   titanium dioxide - 18%
   polydextrose - 7%
   triethyl citrate - 3%
   microcrystalline cellulose - 10%
   talc - 10%
   riboflavin - 5%

It has been found that it is possible to use a much lower concentration of dyes to achieve the same colour intensity.

### Example 4.

The following compositions, which were subsequently used to coat the following products were prepared according to Example 1.

A composition based on HPMC polymer intended for coating a dietary supplement containing magnesium lactate.
HPMC - 45%
magnesium phosphate - 30%
polydextrose - 10%
polyethylene glycol - 10%
talc - 5%

A composition based on HPMC polymer intended for coating a dietary supplement containing a multivitamin core.
HPMC - 50%
magnesium phosphate - 32%
stearic acid - 7%
talc - 3%
beta-cyclodextrin - 4.9%
triethyl citrate - 3%
Tween 80 - 0.1%

A composition based on HPMC polymer intended for coating a dietary supplement containing magnesium citrate and calcium carbonate.
HPMC - 45%
magnesium phosphate - 30%
polyethylene glycol - 15%
microcrystalline cellulose - 10%

A composition based on HPMC polymer intended for coating tablet cores containing melatonin. HPMC - 47%
magnesium phosphate - 30%
polyethylene glycol - 7%
microcrystalline cellulose - 4.9%
Tween 80 - 0.2%
talc - 10%
Indigo carmine aluminium lake - 0.9%

A composition based on HPMC polymer intended for coating tablet cores with a composition containing vitamin C.
HPMC - 47%
magnesium phosphate with a particle size of 60 micrometres - 35%
triethyl citrate - 3%
microcrystalline cellulose - 4.1%
talc - 10%
riboflavin - 0.9%

A barrier composition for tablets (protection against moisture) based on a hydroxyethyl cellulose polymer, intended for coating a dietary supplement containing potassium ions. ethylcellulose - 10%
magnesium phosphate with a particle size of 20 micrometres - 50%
PVP -10%
stearic acid - 5%
dextrose - 25%

A composition based on methacrylic acid, enteric, barrier for tablets (protection against acidic environment), intended for coating a dietary supplement containing a dietary supplement containing *Lactobacillus casei* bacteria.
methacrylic acid copolymer - 40%
talc - 20%
magnesium phosphate with a particle size of 80 micrometers - 32%
sodium bicarbonate - 2%
Tween 80 - 1%

A composition based on PVA polymer intended for coating tablet cores containing valerian and hop extract.
hydrolysed PVA - 40%
magnesium phosphate with a particle size of 40 micrometres - 30%
polyethylene glycol - 15.2%
Talc - 14.8%

A sugar coating system based on a polymer of acacia gum intended for coating chewing gum. acacia gum - 38%
magnesium phosphate - 32%
xylitol - 20%
mannitol - 9.6%
sodium saccharin - 0.05%
flavour - 0.35

## Claims

1. Use of magnesium phosphate as a colouring and opacifying agent in coating compositions for pharmaceuticals, dietary supplements and food products.

2. The use according to claim 1, **characterised in that** the magnesium phosphate composition is used to achieve a weight gain of the coated product from 3 to 7 wt %.

3. The use according to claim 1, **characterised in that** magnesium phosphate with a fineness from 2 to 100 µm is applied.

4. The use according to claim 1, **characterised in that** tribasic magnesium phosphate is applied.

5. The use according to claim 1, **characterised in that** magnesium phosphate is used in a composition comprising this magnesium phosphate at an amount from 0.5 wt % to 80 wt %, and a film-forming substance selected from: polyvinyl alcohol (PVA), hydroxypropyl methylcellulose (HPMC), hydroxypropyl cellulose (HPC), agar, xanthan gum, acacia gum, shellac, gelatin, polyvinylpyrrolidone (PVP), sodium carboxymethyl cellulose, ethyl cellulose, sodium starch gluconate, other starch and cellulose derivatives, pectins or their derivatives, polymers of methacrylic acid at an amount from 40 wt % to 95.5 wt %, wherein a proportion of composition components refers to the dry weight of the composition.

6. The use according to claim 5, **characterised in that** magnesium phosphate is used in a composition comprising, in addition to magnesium phosphate and a film-forming substance, also at least one known excipient selected from: plasticisers, fillers, anti-adhesive substances, anti-caking agents, additional opacifiers, dyes other than white.

7. The use according to claim 6, **characterised in that** magnesium phosphate is used in the composition containing a plasticizer selected from the group comprising: MTCs, vegetable oils, polysorbates (tweens), fatty acids, polyethylene glycols, sorbitol, glycerin, propylene glycol, trimethylaminoethanolamine, triacetin, triethyl citrate, lecithin, or combinations of these substances.

8. The use according to claim 7, **characterised in that** magnesium phosphate is applied in the composition containing a plasticizer at an amount from 0.5 wt % to 20 wt %.

9. The use according to claim 1, **characterised in that** magnesium phosphate is applied in the form of a suspension in water of a composition containing this magnesium phosphate.

10. The use according to claim 7, **characterised in that** an aqueous suspension containing from 10 to 25 wt % of the composition with magnesium phosphate is applied.

11. The use according to claim 1, **characterised in that** magnesium phosphate is used in the compositions for coating tablets, pellets, dragees and similar forms of pharmaceuticals, foodstuffs and dietary supplements requiring coating.

12. A coloring composition in its dry form for use in the coating process of pharmaceuticals, food supplements and food products, containing a film-forming substance and a white dye, **characterised in that** it contains magnesium phosphate as a white dye at an amount from 0.5 wt % to 80% wt %, and the film-forming substance is selected from: polyvinyl alcohol (PVA), hydroxypropyl methylcellulose (HPMC), hydroxypropyl cellulose (HPC), agar, xanthan gum, acacia gum, shellac, gelatin, polyvinylpyrrolidone (PVP), sodium carboxymethyl cellulose, ethyl cellulose, sodium starch gluconate, other starch and cellulose derivatives, pectins or their derivatives, polymers of methacrylic acid at an amount from 20 wt % to 95.5 wt %.

13. A composition according to claim 12, **characterised in that** it contains magnesium phosphate with a fineness from 2 to 100 µm.

14. A composition according to claim 12, **characterised in that** it additionally contain known excipients selected from: plasticisers, fillers, anti-adhesive substances, anti-caking agents, additional opacifiers, dyes other than white.

15. A composition according to claim 14, **characterised in that** the plasticizers are selected from the group comprising: MTCs, vegetable oils, polysorbates (tweens), fatty acids, polyethylene glycols, sorbitol, glycerin, propylene glycol, trimethylaminoethanolamine, triacetin, triethyl citrate, lecithin, or combinations of these substances.

16. A composition according to claim 14 **characterised in that** it contains a plasticiser at an amount from 0.5 wt % to 20 wt %.
